(19) Europäisches Patentamt
European Patent Office
Office européen des brevets

(11) EP 4 793 279 A1

(12) **EUROPEAN PATENT APPLICATION**
published in accordance with Art. 153(4) EPC

(43) Date of publication:
**19.08.2026 Bulletin 2026/34**

(21) Application number: **24876691.7**

(22) Date of filing: **12.10.2024**

(51) International Patent Classification (IPC):
***C07J 19/00** (2006.01)* ***A61K 31/56** (2006.01)*
***A61P 17/00** (2006.01)*

(52) Cooperative Patent Classification (CPC):
**A61K 31/56; A61P 17/00; C07J 19/00**

(86) International application number:
**PCT/CN2024/124481**

(87) International publication number:
**WO 2025/077886 (17.04.2025 Gazette 2025/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.10.2023 CN 202311331585**

(71) Applicant: **EnKang Pharmaceuticals (Guangzhou), Ltd.**
**Guangzhou, Guangdong 511400 (CN)**

(72) Inventors:
- **LIANG, Chun**
  **Guangzhou, Guangdong 511400 (CN)**
- **XU, Jin**
  **Guangzhou, Guangdong 511400 (CN)**
- **MA, Xiang**
  **Guangzhou, Guangdong 511400 (CN)**
- **JIAO, Xueting**
  **Guangzhou, Guangdong 511400 (CN)**

(74) Representative: **Murgitroyd & Company**
**165-169 Scotland Street**
**Glasgow G5 8PL (GB)**

# (54) COMPOUND AND METHOD FOR TREATING AND/OR PREVENTING ACTINIC KERATOSIS

(57) The present application relates to 17β-neriifolin or a pharmaceutically acceptable salt, racemate, stereoisomer, enantiomer, diastereomer, tautomer, metabolite, solvate or prodrug thereof, and a method thereof for treating and/or preventing actinic keratosis (Ak). The compound can penetrate through the skin and act directly on lesion sites, and has an improved efficacy and a relatively high safety.

EP 4 793 279 A1

## Description

The present application claims priority and benefits to the patent application No.202311331585.0 filed with the China National Intellectual Property Administration on October 13, 2023, which is incorporated herein by reference in its entirety.

## TECHNICAL FIELD

The present application relates to the technical field of pharmaceuticals, and particularly to a compound and method for treating and/or preventing actinic keratosis.

## BACKGROUND OF THE INVENTION

It has been reported that neriifolin can target beclin1, inhibit the formation of LC3-associated phagosomes, and ameliorate the progression of experimental autoimmune encephalomyelitis (EAE). Patent No. CN 104736157 A documents that 17β-neriifolin can induce cell cycle arrest and apoptosis in human liver cancer HepG2 cells.

Actinic keratosis (AK) or solar keratosis is a growth (lesion) of squamous scleroderma caused by damage from solar ultraviolet radiation. It mostly occurs in exposed areas such as face, ears, back of the hand, and the like, with the main symptoms being red, brown, and grayish white keratinized patches accompanied by adhesive scales on the surface. As the illness progresses, it seriously affects the patients' appearance and the safety of life. In recent years, the number of AK patients has gradually increased, and it is of great significance to adopt safe and effective methods to treat AK.

The existing treatment methods include cryotherapy, surgical resection, photodynamic therapy, local drug therapy, chemical exfoliation, etc. Cryotherapy uses liquid nitrogen to destroy individual lesions; the main disadvantages of this treatment are inability to treat subclinical lesions and the high recurrence rates, which can be up to 96% within one year. The therapeutic effect of photodynamic therapy is influenced by various factors such as the severity of skin lesions, the type of light source used, the duration of photosensitizer encapsulation, and serum vitamin D levels, and there are also adverse reactions such as pain, erythema, blisters, and scabs. Drug therapy is an optional approach for treating AK and can be combined with other methods to enhance the therapeutic effect on AK. Among them, 5-Fluorouracil, Imiquimod, Butanediol Methanesulfonate and Tirbanibulin have been used in the treatment of actinic keratosis, but the therapeutic effects are limited.

In view of this, drugs suitable for treating actinic keratosis still need to be developed.

## SUMMARY

In one aspect, the present application relates, at least in part, to a compound, which is 17β-neriifolin or a pharmaceutically acceptable salt, racemate, stereoisomer, enantiomer, diastereomer, tautomer, metabolite, solvate or prodrug thereof, for use in the treatment and/or prevention of actinic keratosis via topical administration.

In one aspect, the present application relates, at least in part, to a method for treating and/or preventing actinic keratosis, which comprises administering 17β-neriifolin or a pharmaceutically acceptable salt, racemate, stereoisomer, enantiomer, diastereomer, tautomer, metabolite, solvate or prodrug thereof.

In one aspect, the present application relates, at least in part, to the method for treating and/or preventing actinic keratosis, which comprises administering to a subject in need thereof a therapeutically effective amount of 17β-neriifolin or a pharmaceutically acceptable salt, racemate, stereoisomer, enantiomer, diastereomer, tautomer, metabolite, solvate or prodrug thereof.

In one aspect, 17β-neriifolin or a pharmaceutically acceptable salt, racemate, stereoisomer, enantiomer, diastereomer, tautomer, metabolite, solvate or prodrug thereof is administered to the affected area(s) of the subject(s) at a dose of: about 0.0003 mg to about 100 mg; about 0.008 mg to about 90 mg; about 0.1 mg to about 80 mg; about 0.1 mg to about 70 mg; about 0.1 mg to about 60 mg; about 0.1 mg to about 50 mg; about 0.1 mg to about 40 mg; about 0.1 mg to about 30 mg; about 0.1 mg to about 20 mg; about 0.1 mg to about 15 mg; about 0.1 mg to about 10 mg; about 0.1 mg to about 8 mg; about 0.2 mg to about 5 mg; or about 0.5 mg to about 2.5 mg.

In one aspect, 17β-neriifolin or a pharmaceutically acceptable salt, racemate, stereoisomer, enantiomer, diastereomer, tautomer, metabolite, solvate or prodrug thereof is administered to the affected area(s) of the subject(s) at a dose of: about 0.02 mg, about 0.05 mg, about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3 mg, about 4 mg, or about 5 mg.

In one aspect, 17β-neriifolin or a pharmaceutically acceptable salt, racemate, stereoisomer, enantiomer,

diastereomer, tautomer, metabolite, solvate or prodrug thereof is administered to the affected area(s) of the subject(s) at a dose of: about 0.0003 mg/cm$^2$ to about 10 mg/cm$^2$; preferably about 0.001 mg/cm$^2$ to about 1 mg/cm$^2$; preferably about 0.002 mg/cm$^2$ to about 0.5 mg/cm$^2$; preferably about 0.004 mg/cm$^2$ to about 0.25 mg/cm$^2$; preferably about 0.008 mg/cm$^2$ to about 0.2 mg/cm$^2$; preferably about 0.01 mg/cm$^2$ to about 0.18 mg/cm$^2$; preferably about 0.016 mg/cm$^2$ to about 0.16 mg/cm$^2$; preferably about 0.02 mg/cm$^2$ to about 0.15 mg/cm$^2$; preferably about 0.025 mg/cm$^2$ to about 0.14 mg/cm$^2$; preferably about 0.027 mg/cm$^2$ to about 0.13 mg/cm$^2$; preferably about 0.03 mg/cm$^2$ to about 0.125 mg/cm$^2$; preferably about 0.035 mg/cm$^2$ to about 0.12 mg/cm$^2$; preferably about 0.04 mg/cm$^2$ to about 0.1 mg/cm$^2$.

In one aspect, 17β-neriifolin or a pharmaceutically acceptable salt, racemate, stereoisomer, enantiomer, diastereomer, tautomer, metabolite, solvate or prodrug thereof is administered to the affected area(s) of the subject(s) at a dose of: about 0.001 mg/cm$^2$, about 0.002 mg/cm$^2$, about 0.003 mg/cm$^2$, about 0.004 mg/cm$^2$, about 0.005 mg/cm$^2$, about 0.006 mg/cm$^2$, about 0.007 mg/cm$^2$, about 0.008 mg/cm$^2$, about 0.009 mg/cm$^2$, about 0.01 mg/cm$^2$, about 0.015 mg/cm$^2$, about 0.02 mg/cm$^2$, about 0.025 mg/cm$^2$, about 0.03 mg/cm$^2$, about 0.035 mg/cm$^2$, about 0.04 mg/cm$^2$, about 0.045 mg/cm$^2$, about 0.05 mg/cm$^2$, about 0.055 mg/cm$^2$, about 0.06 mg/cm$^2$, about 0.065 mg/cm$^2$, about 0.07 mg/cm$^2$, about 0.075 mg/cm$^2$, about 0.08 mg/cm$^2$, about 0.085 mg/cm$^2$, about 0.09 mg/cm$^2$, about 0.095 mg/cm$^2$, about 0.1 mg/cm$^2$, about 0.15 mg/cm$^2$, about 0.2 mg/cm$^2$, about 0.25 mg/cm$^2$, about 0.3 mg/cm$^2$, about 0.35 mg/cm$^2$, about 0.4 mg/cm$^2$, about 0.45 mg/cm$^2$, about 0.5 mg/cm$^2$, about 0.55 mg/cm$^2$, about 0.6 mg/cm$^2$, or about 0.65 mg/cm$^2$.

In one aspect, the affected area(s) of the subject(s) is/are about 0.01 cm$^2$ to about 300 cm$^2$; preferably, the affected area(s) of the subject(s) is/are: about 1 cm$^2$ to about 200 cm$^2$, about 1 cm$^2$ to about 100 cm$^2$, about 1 cm$^2$ to about 75 cm$^2$, about 1 cm$^2$ to about 50 cm$^2$, or about 1 cm$^2$ to about 25 cm$^2$; about 10 cm$^2$ to about 200 cm$^2$, about 10 cm$^2$ to about 100 cm$^2$, about 10 cm$^2$ to about 75 cm$^2$, about 10 cm$^2$ to about 50 cm$^2$, or about 10 cm$^2$ to about 25 cm$^2$; about 25 cm$^2$ to about 200 cm$^2$, about 25 cm$^2$ to about 100 cm$^2$, or about 25 cm$^2$ to about 75 cm$^2$; about 25 cm$^2$ to about 90 cm$^2$, about 25 cm$^2$ to about 80 cm$^2$, or about 25 cm$^2$ to about 70 cm$^2$, about 25 cm$^2$ to about 60 cm$^2$, about 25 cm$^2$ to about 50 cm$^2$, about 25 cm$^2$ to about 40 cm$^2$, or about 25 cm$^2$ to about 30 cm$^2$. Preferably, the affected area(s) of the subject(s) is/are about 0.2 cm$^2$, about 0.5 cm$^2$, about 1 cm$^2$, about 2 cm$^2$, about 3 cm$^2$, about 4 cm$^2$, about 5 cm$^2$, about 6 cm$^2$, about 7 cm$^2$, about 8 cm$^2$, about 9 cm$^2$, about 10 cm$^2$, about 11 cm$^2$, about 12 cm$^2$, about 13 cm$^2$, about 14 cm$^2$, about 15 cm$^2$, about 16 cm$^2$, about 17 cm$^2$, about 18 cm$^2$, about 19 cm$^2$, about 20 cm$^2$, about 25 cm$^2$, about 30 cm$^2$, about 35 cm$^2$, about 40 cm$^2$, about 45 cm$^2$, about 50 cm$^2$, about 55 cm$^2$, about 60 cm$^2$, about 65 cm$^2$, about 70 cm$^2$, about 75 cm$^2$, about 80 cm$^2$, about 85 cm$^2$, about 90 cm$^2$, about 95 cm$^2$, or about 100 cm$^2$.

In one aspect, the affected area(s) of the subject(s) is/are on the skin.

In one aspect, the affected area(s) of the subject(s) is/are located at one or more positions independently selected from the scalp, forehead, forearm, face, nose, ears, eyelids, lips, neck, arms, hands, trunk, legs, and feet.

In one aspect, the subject has more than one affected area. In one aspect, 17β-neriifolin or a pharmaceutically acceptable salt, racemate, stereoisomer, enantiomer, diastereomer, tautomer, metabolite, solvate or prodrug thereof is administered to the patient via topical administration.

In one aspect, 17β-neriifolin or a pharmaceutically acceptable salt, racemate, stereoisomer, enantiomer, diastereomer, tautomer, metabolite, solvate or prodrug thereof is formulated into a pharmaceutical composition suitable for topical use.

In one aspect, the content of 17β-neriifolin is about 0.025 wt% to 4 wt%; preferably about 0.05 wt% to 3 wt%; preferably about 0.2 wt% to 0.8 wt%; preferably about 0.4 wt% to 0.8 wt%; preferably about 0.075 wt%, about 0.1 wt%, about 0.125 wt%, about 0.15 wt%, about 0.175 wt%, about 0.2 wt%, about 0.225 wt%, about 0.25 wt%, about 0.275 wt%, about 0.3 wt%, about 0.325 wt%, about 0.35 wt%, about 0.375 wt%, about 0.4 wt%, about 0.425 wt%, about 0.45 wt%, about 0.475 wt%, about 0.5 wt%, about 0.525 wt%, about 0.55 wt%, about 0.575 wt%, about 0.6 wt%, about 0.625 wt%, about 0.65 wt%, about 0.675 wt%, about 0.7 wt%, about 0.725 wt%, about 0.75 wt%, about 0.775 wt%, about 0.8 wt%, about 0.825 wt%, about 0.85 wt%, about 0.875 wt%, about 0.9 wt%, about 0.925 wt%, about 0.95 wt%, about 0.975 wt%, about 1 wt%, about 1.25 wt%, about 1.5 wt%, about 1.75 wt%, about 2.0 wt%, about 2.25 wt%, about 2.5 wt%, or about 2.75 wt%, based on the total weight of the pharmaceutical composition.

In one aspect, the pharmaceutical composition is a gel.

In one aspect, the pharmaceutically acceptable excipients in the gel optionally include a gel matrix. The gel matrix is selected from at least one of sodium carboxymethyl cellulose, carbomer, gelatin, and alginate. In one aspect, the content of the gel matrix is about 0.5 wt% to 5 wt%; preferably about 1 wt% to 4 wt%; preferably about 1 wt% to 3 wt%; more preferably about 1.5 wt%, about 2 wt%, about 2.5 wt%, or about 3 wt%, based on the total weight of the pharmaceutical composition.

In one aspect, the pharmaceutically acceptable excipients in the gel optionally include a humectant. The humectant is selected from at least one of alcoholic humectants; preferably, the humectant is glycerol. In one aspect, the content of the humectant is about 5 wt% to 35 wt%; preferably about 10 wt% to 30 wt%; preferably about 15 wt% to 25 wt%; more preferably about 15 wt%, about 20 wt%, about 25 wt%, or about 30 wt%, based on the total weight of the pharmaceutical composition.

In one aspect, the pharmaceutically acceptable excipients in the gel optionally include a solvent; preferably, the

solvent is selected from at least one of ethanol, propylene glycol, and benzyl alcohol; the ethanol is 95% ethanol or anhydrous ethanol. In one aspect, the content of the solvent is about 20 wt% to 75 wt%; preferably about 25 wt% to 70 wt%; more preferably about 30 wt%, about 35 wt%, about 40 wt%, about 45 wt%, about 50 wt%, about 55 wt%, about 60 wt%, or about 65 wt%, based on the total weight of the pharmaceutical composition.

In one aspect, the pharmaceutically acceptable excipients in the gel optionally include water, preferably purified water, distilled water, or deionized water. In one aspect, the content of the water is about 20 wt% to 75 wt%; preferably about 25 wt% to 70 wt%; more preferably about 30 wt%, about 35 wt%, about 40 wt%, about 45 wt%, about 50 wt%, about 55 wt%, about 60 wt%, or about 65 wt%, based on the total weight of the pharmaceutical composition.

In one aspect, the pharmaceutically acceptable excipients in the gel optionally include a penetration enhancer. In one aspect, the penetration enhancer is selected from at least one of azone, borneol, Tween 80, sodium dodecyl sulfate, and poloxamer; more preferably, the penetration enhancer is azone or borneol. In one aspect, the content of the penetration enhancer is about 0 wt% to 5 wt%; preferably about 0.1 wt% to 4 wt%; more preferably about 0.25 wt%, about 0.5 wt%, about 0.75 wt%, about 1 wt%, about 1.25 wt%, about 1.5 wt%, about 1.75 wt%, about 2 wt%, about 2.25 wt%, about 2.5 wt%, about 2.75 wt%, about 3 wt%, about 3.25 wt%, about 3.5 wt%, or about 3.75 wt%, based on the total weight of the pharmaceutical composition.

In one aspect, the pharmaceutically acceptable excipients optionally include a preservative. The preservative is a paraben preservative and/or butylated hydroxytoluene; preferably, the paraben preservative is methylparaben and/or ethylparaben. In one aspect, the content of the preservative is about 0 wt% to 4 wt%; preferably about 0.1 wt% to 3 wt%; more preferably about 0.25 wt%, about 0.5 wt%, about 0.75 wt%, about 1 wt%, about 1.25 wt%, about 1.5 wt%, about 1.75 wt%, about 2 wt%, about 2.25 wt%, about 2.5 wt%, or about 2.75 wt%, based on the total weight of the pharmaceutical composition.

In one aspect, the pharmaceutical composition comprises:

about 0.2 wt% to 0.8 wt% 17β-neriifolin or a pharmaceutically acceptable salt, racemate, stereoisomer, enantiomer, diastereomer, tautomer, metabolite, solvate or prodrug thereof; and/or
about 1.5 wt% gel matrix; and/or
about 40 wt% solvent; and/or
about 0.5 wt% penetration enhancer; and/or
about 20 wt% humectant;
the balance being water.

In one aspect, the pharmaceutical composition is a topical composition comprising 17β-neriifolin free base, wherein the content of 17β-neriifolin free base, as determined by HPLC, is greater than about 90%, preferably greater than about 90.5%, greater than about 91%, greater than about 91.5%, greater than about 92%, greater than about 92.5%, greater than about 93%, greater than about 93.5%, greater than about 94%, greater than about 94.5%, greater than about 95%, greater than about 95.5%, greater than about 96%, greater than about 96.5%, greater than about 97%, greater than about 97.5%, greater than about 98%, greater than about 98.5%, greater than about 99%, greater than about 99.5%, greater than about 99.6%, greater than about 99.7%, greater than about 99.8%, or greater than about 99.9%.

In one aspect, the dosage form of the topical composition includes a powder, a spray, an ointment, an emulgel, a paste, a cream, a lotion, a gel, a solution, a patch, and an inhalant.

In one aspect, the dosage form of the topical composition includes a gel.

In one aspect, the amount of the pharmaceutical composition administered to an affected skin area(s) ranges/range from about 0.001 g/cm$^2$ of skin surface area to about 0.5 g/cm$^2$ of skin surface area; preferably about 0.0015 g/cm$^2$ of skin surface area to about 0.2 g/cm$^2$ of skin surface area; preferably about 0.0015 g/cm$^2$ of skin surface area to about 0.15 g/cm$^2$ of skin surface area; preferably about 0.002 g/cm$^2$ of skin surface area to about 0.125 g/cm$^2$ of skin surface area; preferably about 0.003 g/cm$^2$ of skin surface area to about 0.1 g/cm$^2$ of skin surface area; preferably about 0.004 g/cm$^2$ of skin surface area; about 0.005 g/cm$^2$ of skin surface area; about 0.006 g/cm$^2$ of skin surface area; about 0.007 g/cm$^2$ of skin surface area; about 0.008 g/cm$^2$ of skin surface area; about 0.009 g/cm$^2$ of skin surface area; about 0.01 g/cm$^2$ of skin surface area; about 0.0125 g/cm$^2$ of skin surface area; about 0.015 g/cm$^2$ of skin surface area; about 0.0175 g/cm$^2$ of skin surface area; about 0.02 g/cm$^2$ of skin surface area; about 0.03 g/cm$^2$ of skin surface area; about 0.04 g/cm$^2$ of skin surface area; about 0.05 g/cm$^2$ of skin surface area; about 0.06 g/cm$^2$ of skin surface area; about 0.07 g/cm$^2$ of skin surface area; about 0.08 g/cm$^2$ of skin surface area; or about 0.09 g/cm$^2$ of skin surface area.

In one aspect, 17β-neriifolin or a pharmaceutically acceptable salt, racemate, stereoisomer, enantiomer, diastereomer, tautomer, metabolite, solvate or prodrug thereof is administered daily, at one-day intervals, at two-day intervals, at three-day intervals, at four-day intervals, at five-day intervals, at six-day intervals, or at seven-day intervals. Generally, on the day of administration, one, two, three, or four administrations are performed.

In one aspect, 17β-neriifolin or a pharmaceutically acceptable salt, racemate, stereoisomer, enantiomer, diastereomer, tautomer, metabolite, solvate or prodrug thereof is administered once a week, once every six days, once

every five days, once every four days, once every three days, once every two days, once a day, twice a day, three times a day, or four times a day; preferably once every two days or once a day.

In one aspect, the administration cycle of 17β-neriifolin or a pharmaceutically acceptable salt, racemate, stereoisomer, enantiomer, diastereomer, tautomer, metabolite, solvate or prodrug thereof may be 1 to 100 days, preferably 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 days.

In one aspect, 17β-neriifolin or a pharmaceutically acceptable salt, racemate, stereoisomer, enantiomer, diastereomer, tautomer, metabolite, solvate or prodrug thereof may be administered at a frequency of once or twice daily continuously for more than one day per week, followed by discontinuation of the administration for the rest of the week. In one aspect, the administration is performed once or twice daily every other day. In one aspect, the administration is performed once or twice daily every three, four, five, six, or seven days. In one aspect, the administration is performed once or twice daily for two days in a row every three, four, five, six, or seven days. In one aspect, the administration is performed once or twice daily for three days in a row every four, five, six, or seven days. In one aspect, the administration is performed once or twice daily for four days in a row every five, six, or seven days. In one aspect, the administration is performed until actinic keratosis is completely cleared. In one aspect, the administration is topical administration.

In one aspect, the drug is kept in contact with the skin for about 0.5 h to about 24 hrs, optionally about 1 h, about 1.5 hrs, about 2 hrs, about 2.5 hrs, about 3 hrs, about 3.5 hrs, about 4 hrs, about 4.5 hrs, about 5 hrs, about 5.5 hrs, about 6 hrs, about 6.5 hrs, about 7 hrs, about 7.5 hrs, about 8 hrs, about 8.5 hrs, about 9 hrs, about 9.5 hrs, about 10 hrs, about 11 hrs, about 12 hrs, about 13 hrs, about 14 hrs, about 15 hrs, about 16 hrs, about 17 hrs, about 18 hrs, about 19 hrs, about 20 hrs, about 21 hrs, about 22 hrs, about 23 hrs, or about 24 hrs per administration.

In one aspect, compared to other treatments of actinic keratosis, the administration of 17β-neriifolin or a pharmaceutically acceptable salt, racemate, stereoisomer, enantiomer, diastereomer, tautomer, metabolite, solvate or prodrug thereof reduces the number and/or severity of local skin reactions or other adverse effects in the subject(s).

In one aspect, compared to other treatments of actinic keratosis, the administration of 17β-neriifolin or a pharmaceutically acceptable salt, racemate, stereoisomer, enantiomer, diastereomer, tautomer, metabolite, solvate or prodrug thereof reduces the number of subjects experiencing local skin reactions or other adverse effects.

In one aspect, the local skin reaction is selected from vesicle formation, pustule formation, erosion, ulceration, redness, swelling, scaling, desquamation, induration, dryness, pus, blistering, and the like.

In one aspect, the other side effect is selected from pain at the site of administration, pruritus at the site of administration, irritation at the site of administration, swelling at the site of administration, burning sensation at the site of administration, infection at the site of administration, edema around the eyes, nasopharyngitis, chills, sore throat, blepharoptosis, eye puffiness, hypopigmentation, hyperpigmentation, headache, and the like.

In one aspect, the present application relates, at least in part, to 17β-neriifolin or a pharmaceutically acceptable salt, racemate, stereoisomer, enantiomer, diastereomer, tautomer, metabolite, solvate or prodrug thereof for use (e.g., topical use) in the treatment and/or prevention of actinic keratosis. In some aspects, 17β-neriifolin or a pharmaceutically acceptable salt, racemate, stereoisomer, enantiomer, diastereomer, tautomer, metabolite, solvate or prodrug thereof is used at the dose, on the administration schedule, and/or in one or more affected areas of the subject(s) in need thereof, as described herein. In one aspect, the present application relates, at least in part, to use (e.g., topical use) of 17β-neriifolin or a pharmaceutically acceptable salt, racemate, stereoisomer, enantiomer, diastereomer, tautomer, metabolite, solvate or prodrug thereof in the treatment and/or prevention of actinic keratosis. In some aspects, 17β-neriifolin or a pharmaceutically acceptable salt, racemate, stereoisomer, enantiomer, diastereomer, tautomer, metabolite, solvate or prodrug thereof is used at the dose, on the administration schedule, and/or in one or more affected areas of the subject(s) in need thereof, as described herein. In one aspect, the present application relates, at least in part, to use of 17β-neriifolin or a pharmaceutically acceptable salt, racemate, stereoisomer, enantiomer, diastereomer, tautomer, metabolite, solvate or prodrug thereof in the preparation of a medicament for treating and/or preventing actinic keratosis. In some aspects, 17β-neriifolin or a pharmaceutically acceptable salt, racemate, stereoisomer, enantiomer, diastereomer, tautomer, metabolite, solvate or prodrug thereof is used at the dose, on the administration schedule, and/or in one or more affected areas of the subject(s) in need thereof, as described herein. In one aspect, the present application relates, at least in part, to a therapeutic agent for actinic keratosis, comprising 17β-neriifolin or a pharmaceutically acceptable salt, racemate, stereoisomer, enantiomer, diastereomer, tautomer, metabolite, solvate or prodrug thereof. In some aspects, 17β-neriifolin or a pharmaceutically acceptable salt, racemate, stereoisomer, enantiomer, diastereomer, tautomer, metabolite, solvate or prodrug thereof is used at the dose, on the administration schedule, and/or in one or more affected areas of the subject(s) in need thereof, as described herein.

Actinic keratosis (AK) is a common skin disease caused by excessive exposure to ultraviolet radiation. AK is a rough, dry, yellow brown, pink, or red scar (lesion) that often appears on the head (including the face, throat, neck, nose, forehead, ears, or lips). AK may also appear on other body parts that receive prolonged exposure to strong sunlight, such as the hands, back, and other areas of the torso and legs.

The term "trunk" as used herein refers to a part of a subject excluding the arms, legs, and head.

AK is most commonly found in middle-aged or elderly people with very fair skin. Subjects with AK may have a single lesion or multiple lesions.

The clinical variants of AK include: traditional (or common) AK, hypertrophic (or hyperkeratotic) AK, atrophic AK, AK with skin angle, pigmented AK, actinic cheilitis, etc. Unless otherwise specified, the methods described herein are applicable to all clinical variants, including those listed in this article.

Treatment of AK includes cryosurgery, surgical resection and/or curettage of diseased areas, photodynamic therapy, and topical preparations (such as cream, gel, patch, etc.) containing steroids, Fluorouracil, Diclofenac, Imiquimod, and 5-Aminolevulinic acid.

The approved treatment of AK is Picato, a gel containing Ingenol-3-angelate (0.015% or 0.05%). The gel is applied once a day (0.015%) to the diseased area(s) on the face or scalp for three consecutive days or once a day (0.05%) to the diseased area(s) on the trunk or limbs for two consecutive days.

It is known that the skin toxicity associated with the use of other AK treatments, such as Picato, produces undesirable side effects or adverse reactions, namely local skin reactions (LSR), including vesicle formation, pustule formation, erosion, ulceration, redness, swelling, peeling, flaking, hard lumps, dryness, pus, and blisters. Other side effects include pain, pruritus, irritation, swelling, burning sensation, infection, periocular edema, nasopharyngitis, chills, sore throat, drooping eyelids, eye swelling, hypopigmentation, hyperpigmentation, and headache at the application sites.

The phrase "until actinic keratosis is cleared" as used herein refers to a condition in which the lesion(s) on a subject having AK have substantially or completely disappeared from the treated area(s) of the subject. In one embodiment, "substantially" in this context refers to the disappearance of more than 50% of AK from the treated area(s) of the subject. In another embodiment, "substantially" refers to the disappearance of more than 60% of AK from the treated area(s) of the subject. In another embodiment, "substantially" refers to the disappearance of more than 70% of AK from the treated area(s) of the subject. In another embodiment, "substantially" refers to the disappearance of more than 80% of AK from the treated area(s) of the subject. In another embodiment, "substantially" refers to the disappearance of more than 90% of AK from the treated area(s) of the subject. In another embodiment, "substantially" refers to the disappearance of more than 95% of AK from the treated area(s) of the subject. In another embodiment, "substantially" refers to the disappearance of more than 99% of AK from the treated area(s) of the subject.

As used throughout the present disclosure, the singular forms "a," "an," and "the" include plural referents, unless otherwise clearly indicated in the context. Thus, for example, reference to "a method" includes multiple such methods, and reference to "a dose" includes reference to one or more doses and equivalents thereof known to those skilled in the art, and so forth.

The term "comprising" is intended to mean that the method includes the enumerated elements but does not exclude other elements. "Consisting essentially of...", when used to define a method, shall mean the exclusion of other elements that have any fundamental significance to the combination when used for the intended purpose. Thus, a method consisting essentially of the elements defined herein does not exclude substantial method steps. "Consisting of..." means excluding multiple substantial method steps. Embodiments defined by each of these transitional terms fall within the scope of the present application.

Unless otherwise clearly specified, the terms "approximately" and "about" are synonymous. In one embodiment, "approximately" and "about" refer to ±5%, ±4.5%, ±4%, ±3.5%, ±3%, ±2.5%, ±2%, ±1.75%, ±1.5%, ±1.25%, ±1%, ±0.9%, ±0.8%, ±0.7%, ±0.6%, ±0.5%±0.4%, ±0.3%, ±0.2%, ±0.1%, ±0.09%, ±0.08%, ±0.07%, ±0.06%, ±0.05%, ±0.04%, ±0.03%, ±0.02%, or ±0.01% of the stated amount, value, or duration. In another embodiment, "approximately" and "about" refer to ±2.5%, ±2%, ±1.75%, ±1.5%, ±1.25%, ±1%, ±0.9%, ±0.8%, ±0.7%, ±0.6%, or ±0.5% of the listed amount, value, or duration. In another embodiment, "approximately" and "about" refer to ±1% of the listed amount, value, or duration. In another embodiment, "approximately" and "about" refer to ±0.5% of the listed amount, value, or duration. In another embodiment, "approximately" and "about" refer to 0.1% of the listed amount, value, or duration.

The term "subject" includes any living organism having or at risk of developing actinic keratosis. In one embodiment, the term "subject" refers to mammals having or at risk of developing actinic keratosis. In one embodiment, the term "subject" refers to humans having or at risk of developing actinic keratosis. Unless otherwise clearly indicated, the terms "patient(s)" and "subject(s)" are synonymous.

As used herein, the term "therapeutically effective amount" refers to an amount of an agent (e.g., 17β-neriifolin or a pharmaceutically acceptable salt, racemate, stereoisomer, enantiomer, diastereomer, tautomer, metabolite, solvate or prodrug thereof) that is used for treating, ameliorating, or preventing a diagnosed disease or condition (e.g., AK) or exhibits a detectable therapeutic or inhibitory effect. The effect can be detected by any assay methods known in the art. The exact effective amount for a subject depends on: the subject's body weight, physique, and health status; the nature and severity of the disorder; as well as the therapeutic agent or combination of therapeutic agents selected for administration. The therapeutically effective amount for a given case can be determined through routine experimentation within the skill and judgment of a clinician.

For any compound, the therapeutically effective amount can be estimated in animal models, typically rats, mice, rabbits, dogs, or pigs. Animal models can also be used to determine appropriate concentration ranges and routes of

administration. Such information can then be utilized to determine useful doses and routes of administration in humans. Therapeutic/prophylactic efficacy and toxicity can be determined by standard pharmaceutical procedures in cell cultures or experimental animals, such as determining the ED50 (the dose therapeutically effective in 50% of a population) and LD50 (the dose lethal to 50% of a population). The dose ratio between toxicity and therapeutic effect is the therapeutic index, which can be expressed as the LD50/ED50 ratio. The dose may vary within this range, depending on the dosage form employed and the sensitivity of the subject.

The dose and administration are adjusted to provide sufficient levels of the active ingredient or to maintain the desired effect. Factors that may be considered include the severity of the disease state, location of the disease on the subject, overall health status of the subject, age, body weight, and sex of the subject, diet, time and frequency of administration, pharmaceutical combination(s), response sensitivity, and tolerance/response to treatment. Depending on the half-life and clearance rate, 17β-neriifolin or a pharmaceutically acceptable salt, racemate, stereoisomer, enantiomer, diastereomer, tautomer, metabolite, solvate or prodrug thereof may be administered daily, every other day, every three days, every four days, every five days, every six days, weekly, twice weekly, or once every two weeks.

For any of the methods described herein, 17β-neriifolin or a pharmaceutically acceptable salt, racemate, stereoisomer, enantiomer, diastereomer, tautomer, metabolite, solvate or prodrug thereof may be administered topically, intradermally, interepidermally, intragingivally, intraocularly, nasally, ophthalmically, percutaneously, periodontally, subconjunctivally, sublingually, transmucosally, or aurally. In one embodiment, 17β-neriifolin or a pharmaceutically acceptable salt, racemate, stereoisomer, enantiomer, diastereomer, tautomer, metabolite, solvate or prodrug thereof may be administered topically.

Unless otherwise stated, all percentages and ratios used herein are by weight. The term "17β-neriifolin" as used herein has a molecular formula as follows:

In the medicament, 17β-neriifolin or a pharmaceutically acceptable salt, racemate, stereoisomer, enantiomer, diastereomer, tautomer, metabolite, solvate or prodrug thereof is maintained in a substantially dissolved form. Preferably, the medicament comprises at least 50% of the dissolved form; more preferably, the medicament comprises at least 70% of the dissolved form; more preferably, the medicament comprises at least 90% of the dissolved form; more preferably, the medicament comprises 100% of the dissolved form. Undissolved crystals may be present in any known polymorphic forms. The particle size of 17β-neriifolin or a pharmaceutically acceptable salt, racemate, stereoisomer, enantiomer, diastereomer, tautomer, metabolite, solvate or prodrug thereof may vary from 1 micron to 100 microns (D90) as measured by laser diffraction.

In some embodiments, the present application provides a pharmaceutical composition comprising 17β-neriifolin or a pharmaceutically acceptable salt, racemate, stereoisomer, enantiomer, diastereomer, tautomer, metabolite, solvate or prodrug thereof.

In some embodiments, the pharmaceutical composition is prepared from 17β-neriifolin or a pharmaceutically acceptable salt, racemate, stereoisomer, enantiomer, diastereomer, tautomer, metabolite, solvate or prodrug thereof, and pharmaceutically acceptable excipients, such that the pharmaceutical composition is a medicament suitable for topical use.

The medicament provided according to the present application may take any suitable form, adopting an appropriate form corresponding to the intended use. By way of example and without limitation, the medicament may be formulated as a powder, spray, ointment, paste, cream, lotion, solution (e.g., rinse, suspension), patch, inhalant, gel (e.g., hydrogel), or emulsion for direct topical application. Depending on the dosage form and use, suitable pharmaceutically acceptable excipients may be selected to formulate a topical formulation in the corresponding form, wherein the pharmaceutically acceptable excipients include: vehicles, diluents, solubilizers, emulsifying agents, antioxidants, binders, dispersants, lubricants, preservatives, and the like.

In some embodiments, the pharmaceutical composition is a gel.

In some embodiments, the pharmaceutically acceptable excipients in the gel optionally include a gel matrix. The gel has a large drug-loading capacity, possesses administration dose flexibility, and can adhere to administration sites for a long time, exhibiting a long-lasting and mild effect. In addition, it demonstrates good biocompatibility and stability, and is

easy to apply and clean.

In some embodiments, the gel matrix is selected from at least one of sodium carboxymethyl cellulose, carbomer, gelatin, and alginate. Various types of carbomer can be used, such as Carbomer 934, Carbomer 940, Carbomer 910, or mixtures thereof, with Carbomer 940 being preferred. Carbomer is commercially available, e.g., from BF Goodrich.

In some embodiments, the pharmaceutically acceptable excipients in the gel optionally include a humectant. The humectant can increase the adhesion of the formulation, so that the formulation has relatively good spreadability and viscosity.

The penetration enhancer can increase the rate at which the active ingredient penetrates the skin, delivering the active ingredient to the desired site of action in the epidermis and/or dermis. The penetration enhancer can minimize skin irritation and systemic exposure, while achieving local skin/epidermal delivery. Furthermore, it ensures that the majority of the drug substantially remains localized within the skin (i.e., at the site of action) to treat specific skin disorders, while also reducing systemic toxic side effects.

The term "wt%" as used herein refers to the weight of a component relative to the total components, unless otherwise stated.

In the embodiments of the present application, "treat", "treating", or "treatment" refers to obtaining a desired pharmacological and/or physiological effect. The effect may be prophylactic, so as to completely or partially prevent a disease or a symptom thereof; and/or may be therapeutic, so as to partially or completely cure a disease and/or the adverse effects caused by the disease. "Treat", "treating", or "treatment" used herein encompasses a disease in mammals, particularly in humans, including: inhibiting the disease, e.g., blocking the progression of the disease; or alleviating the disease, e.g., mitigating a symptom associated with the disease. "Treat", "treating", or "treatment" as used herein encompasses any administration of a drug or compound to an individual to treat, cure, alleviate, ameliorate, mitigate, or inhibit a disease in the individual, including but not limited to administration of a medicament comprising the drug described herein to an individual in need thereof.

For the treatment of actinic keratosis, the pharmaceutical composition of the present application may be administered topically to the affected area(s) in any conventional manner known in the art, for example, by means of a dropper, applicator stick, or cotton swab, by means of intradermal or percutaneous patch delivery, or simply by applying the formulation to the skin area(s) with a finger, sponge, pad, or swab.

The pharmaceutical composition may be used in combination with one or more additional methods and drugs for treating actinic keratosis. The pharmaceutical composition described above may be administered to a subject simultaneously or sequentially with other drugs or treatments within a certain time interval, such that the active ingredients or agents can act cooperatively to treat or prevent actinic keratosis.

The compounds decribed herein contain asymmetric centers and therefore may exist as enantiomers. The compounds have multiple asymmetric centers, which additionally allow for their existence as diastereomers. Enantiomers and diastereomers fall within the broader class of stereoisomers. All such possible stereoisomers include substantially pure resolved enantiomers, and racemic mixtures and diastereomeric mixtures thereof. All stereoisomers of the compounds and/or pharmaceutically acceptable salts thereof are encompassed. Unless otherwise stated, reference to one isomer applies to any possible isomers. When the isomeric components are not specified, all possible isomers are included.

A mixture of diastereomers can be separated into individual diastereomers based on their physicochemical differences by methods well known to those skilled in the art, such as chromatography and/or fractional crystallization. Enantiomers can be separated by converting the enantiomeric mixture into a diastereomeric mixture by reaction with a suitable optically active compound (e.g., a chiral auxiliary such as a chiral alcohol or Mosher's acid chloride), then separating the diastereomers, and converting (e.g., hydrolyzing) each diastereomer to the corresponding pure enantiomer. Enantiomers can also be separated using a chiral HPLC column.

"Pharmaceutically acceptable salt" includes, but is not limited to: salts of inorganic acids, selected from, for example, hydrochloride, phosphate, hydrogen phosphate, hydrobromide, sulfate, sulfite, and nitrate; and salts of organic salts, selected from, for example, malate, maleate, fumarate, tartrate, succinate, citrate, lactate, methane sulfonate, p-toluenesulfonate, 2-hydroxyethylsulfonate, benzoate, salicylate, stearate, alkanoate such as acetate, and salts of HOOC-(CH2)n-COOH, where n is selected from 0-4. Similarly, examples of pharmaceutically acceptable cations include, but are not limited to, sodium, potassium, calcium, aluminum, lithium, and ammonium salts.

Additionally, if the compound is obtained as an acid addition salt, the free base can be obtained by basifying a solution of the acid salt. Conversely, if the product is a free base, an addition salt (e.g., a pharmaceutically acceptable addition salt) can be prepared by dissolving the free base in a suitable organic solvent and treating the solution with an acid, consistent with the conventional procedures for preparing an acid addition salt from a basic compound. Those skilled in the art will appreciate a variety of synthetic methods that can be used to prepare non-toxic pharmaceutically acceptable addition salts without undue experimentation.

"Prodrug" refers to a compound that is converted into the parent drug *in vivo.* Prodrugs are often useful because, in some cases, they may be easier to administer than the parent drug. They may be bioavailable, for example, by oral

administration, whereas the parent drug is not. The prodrug may also exhibit improved solubility in a pharmaceutical composition compared to the parent drug. An example (without limitation) of the prodrug is a compound as described herein which is administered as an ester (the "prodrug") to facilitate transport across the cell membrane where water solubility is disadvantageous for this transportation, but which is subsequently metabolically hydrolyzed to a carboxylic acid and an active entity, once inside the cells where water solubility becomes advantageous. A further example of the prodrug may be a short peptide (polyamino acid) linked to an acid group, wherein the peptide can be metabolized to release the active moiety. In certain embodiments, when administered *in vivo,* the prodrug is chemically converted into the biologically, pharmacologically, or therapeutically active form of the compound. In certain embodiments, the prodrug is enzymatically metabolized through one or more steps or processes to yield the biologically, pharmacologically, or therapeutically active form of the compound. To produce a prodrug, a pharmaceutically active compound is modified such that the active compound is regenerated upon *in vivo* administration. The prodrug may be designed to alter the drug's metabolic stability or transport properties to mask side effects or toxicity, thereby improving the effect of the drug or altering other properties or characteristics of the drug. Based on the knowledge of pharmacodynamic methods and *in vivo* drug metabolism, once a pharmaceutically active compound is known, a person skilled in the art can design prodrugs of the compound.

"Metabolite" of the compound disclosed herein is a derivative of the compound that is formed when the compound is metabolized. The term "active metabolite" refers to a biologically active derivative of the compound that is formed when the compound is metabolized. The term "metabolized" as used herein refers to the collective processes by which a particular substance is altered within an organism (including, but not limited to, hydrolysis reactions and enzyme-catalyzed reactions such as oxidation reactions). Thus, enzymes can result in specific structural transformations into new compounds. For example, cytochrome P450 catalyzes various oxidation and reduction reactions, while diphospho-glucuronosyltransferases catalyze the conversion of activated glucuronic acid molecules to aromatic alcohols, aliphatic alcohols, carboxylic acids, amines, and free sulfhydryl groups.

Although specific embodiments of the present disclosure have been illustrated and described, various other changes and modifications may be made without departing from the spirit and scope of the present disclosure. The scope of the appended claims encompasses all such changes and modifications that fall within the scope of the present disclosure.

Other features and advantages of the present application will be apparent from the various embodiments. The provided embodiments are illustrative of various components and methodologies useful in implementing the present application.

These embodiments do not limit the claimed application. Based on the present disclosure, a skilled artisan can identify and employ other components and methodologies that can be used to implement the present application.

## DETAILED DESCRIPTION

The present application will be further illustrated in detail with reference to the following examples. It should be understood that the specific embodiments described herein are merely intended to illustrate, rather than limit, the present disclosure.

It should be noted that the examples and features in the embodiments of the present application may be combined with one another in the absence of conflict.

It should be noted that the endpoints of the ranges and any values disclosed herein are not limited to the precise ranges or values, and these ranges or values should be construed as encompassing values that are close to these ranges or values. The numerical ranges between the endpoint values of each range, between an endpoint value and a single point value of each range, and between two single point values, can be combined with one another to obtain one or more new numerical ranges, which should be construed as being specifically disclosed herein.

The examples without specified techniques or conditions were conducted according to the techniques or conditions described in the literature of the art or according to product instructions. The adopted reagents or instruments without specified manufacturers are all conventional, commercially available products.

The additional aspects and the advantages of the present application will be partially provided in the following description, will partially become apparent from the following description, or will be learned through the practice of the present application.

Example 1

17β-Neriifolin is poorly soluble in water, with a water solubility of about 70 μg/mL. Therefore, the solubility of 17β-neriifolin in organic solvents at different concentrations was investigated. The experimental results are shown in the table below:

| Solvent | Saturation solubility (mg/mL) |
|---|---|
| 10% ethanol | 0.16 |
| 20% ethanol | 0.38 |
| 30% ethanol | 0.91 |
| 40% ethanol | 2.17 |
| 10% propylene glycol | 0.08 |
| 20% propylene glycol | 0.10 |
| 30% propylene glycol | 0.42 |
| 40% propylene glycol | 0.75 |

It can be seen that 17β-neriifolin demonstrated relatively good solubility in ethanol and propylene glycol. Therefore, ethanol and propylene glycol can be used as solvents to dissolve 17β-neriifolin.

Example 2

The solubility of 17β-neriifolin in ethanol at different concentrations was studied (at 20 °C).

An appropriate amount of 17β-neriifolin drug substance was weighed and divided into 3 portions, each placed in a beaker. The first portion was dissolved in anhydrous ethanol and then diluted with water to achieve a final ethanol concentration of 20% and a sample concentration of 8 mg/g, and the diluted solution was observed for the presence of precipitation. The second portion was dissolved in anhydrous ethanol and then diluted with water to achieve final ethanol concentrations of 75%, 66%, 50%, and 40%, corresponding to sample concentrations of 50 mg/g, 40 mg/g, 32 mg/g, and 24 mg/g, respectively, and the diluted solutions were observed for the presence of precipitation. The third portion was dissolved in anhydrous ethanol and then diluted with water to achieve an ethanol concentration of 45% and a sample concentration of 20 mg/g, and the diluted solution was observed for the presence of precipitation.

The results are shown in the table below:

| No. | 17β-Neriifolin (mg) | Anhydrous ethanol (g) | Water (g) | Total solvent amount (g) | Ethanol concentration (%) | Sample concentration (mg/g) | Dissolution behavior |
|---|---|---|---|---|---|---|---|
| 1 | 81 | 2.00 | 8.00 | 10 | 20 | 8 | Dissolved |
| 2 | 201 | 3.98 | 0 | 3.98 | 100 | 50 | Dissolved |
| | | 3.98 | 1.00 | 4.98 | 75 | 40 | Dissolved |
| | | 3.98 | 2.00 | 5.98 | 66 | 32 | Dissolved |
| | | 3.98 | 4.00 | 7.98 | 50 | 24 | Dissolved |
| 3 | 202 | 4.50 | 5.50 | 10.00 | 45 | 20 | Dissolved |

It can be seen that 17β-neriifolin can be dissolved in 20% ethanol at 8 mg/g, in 45% ethanol at 20 mg/g

Example 3

Compatibility test of sodium carboxymethyl cellulose and Carbomer with 17β-neriifolin:
17β-Neriifolin was mixed with sodium carboxymethyl cellulose or Carbomer in a mass ratio of 1:5, and the mixtures were stored for 10 days under the conditions of 60°C±2°C, RH 90%±5%, and a total cumulative illuminance of greater than 1.2 x106 Lux•hr to investigate the impact of the gel matrix on the stability of 17β-neriifolin. The experimental results show that the high temperature, high humidity, and illumination exhibited no impact on the related substances and drug content of 17β-neriifolin combined with sodium carboxymethyl cellulose, and the stability of this combination was significantly superior to that of the combination with Carbomer.

The detailed results of the compatibility test of 17β-neriifolin with sodium carboxymethyl cellulose are shown in the table below.

| | Condition | Labeled amount (%) | Related substances (%) | | | | | | | | | |
|---|---|---|---|---|---|---|---|---|---|---|---|---|
| | | | Impurity D | Impurity E | Impurity A | Impurity B | RRT0.73 | Impurity C | RRT1.10 | Impurity F | RRT2.18 | Total impurities |
| CMC-Na | 0 days | 99.56 | 0.13 | 0.15 | 0.17 | 0.36 | 0.05 | 0.25 | 0.04 | 0.14 | 0.03 | 1.32 |
| | High temperature for 10 days | 100.95 | 0.13 | 0.15 | 0.17 | 0.36 | 0.05 | 0.25 | 0.04 | 0.14 | 0.03 | 1.32 |
| | High humidity for 10 days | 99.42 | 0.13 | 0.15 | 0.17 | 0.36 | 0.05 | 0.25 | 0.04 | 0.14 | 0.03 | 1.32 |
| | Strong light for 10 days | 99.96 | 0.13 | 0.15 | 0.17 | 0.36 | 0.05 | 0.25 | 0.04 | 0.14 | 0.03 | 1.32 |

Example 4

17β-Neriifolin drug substance was weighed and dissolved in anhydrous ethanol aided by ultrasonication and stirring. Azone was added, and the mixture was stirred until uniformity. Water was added, and the mixture was stirred until uniformity. Sodium carboxymethyl cellulose was added, and the mixture was stirred until uniformity. Glycerol was added, and the mixture was stirred until uniformity, and allowed to swell overnight. The mixture was then homogenized to obtain the finished gel product. The physical properties of each gel were scored, and the experimental results are shown in the table below:

| Composition | Formula 1 (%) (%) (%) (%) | Formula 2 (%) | Formula 3 (%) | Formula 4 (%) |
|---|---|---|---|---|
| 17β-Neriifolin | 0.4 | 0.4 | 0.4 | 0.4 |
| Azone | 1.0 | 1.0 | 1.0 | 1.0 |
| Anhydrous ethanol | 40 | 40 | 40 | 40 |
| Glycerol | 20 | 20 | 20 | 20 |
| Sodium carboxymethyl cellulose | 1.0 | 1.5 | 2.0 | 3.0 |
| Making up with water to | 100 | 100 | 100 | 100 |
| Physical property score | 16 | 18 | 17 | 17 |

A total score within the range of 1-10 indicates that the prepared gel has poor physical properties, which may affect medication experience of patients; a total score within the range of 11-15 indicates that the gel is suitable for topical administration with relatively good medication experience; and a total score within the range of 16-20 indicates that the gel possesses good physical properties and is easy to administer without causing any discomfort during administration. It can be seen that when the content of sodium carboxymethyl cellulose is in the range of 1.0-3.0 wt%, the resulting gels have good physical properties.

Example 5

Gel formulations were prepared according to the method in Example 4 to study the impact of different humectant contents on the quality of the gel of the present application. The experimental results are shown in the table below:

| Composition | Formula 5 (%) | Formula 6 (%) | Formula 7 (%) |
|---|---|---|---|
| 17β-Neriifolin | 0.4 | 0.4 | 0.4 |
| Azone | 1.0 | 1.0 | 1.0 |
| Anhydrous ethanol | 40 | 40 | 40 |
| Glycerol | 15 | 20 | 25 |
| CMC-Na | 1.5 | 1.5 | 1.5 |
| Making up with water to | 100 | 100 | 100 |
| Physical property total score | 18 | 18 | 17 |

It can be seen that when the content of glycerol is in the range of 15-25 wt%, the resulting gels have good physical properties.

Example 6

Gels were prepared according to the method in Example 4 to study the skin irritation caused by the gels containing different penetration enhancers and different amounts of the penetration enhancers:
Healthy young pigs (half male and half female) with no back skin injury were used. Comparative administration was performed by topical application on the left and right sides of the same animal's skin, and the administration sites were covered with patches after application. The test gel was administered to the left side, and white vaseline was administered

to the right side as a negative control. The administration area was 3.0 cm × 3.0 cm, and the dose was 20 mg/cm$^2$. The administration was performed once daily. The patches on the administration sites were maintained on both sides of the skin for about 4 hrs before removal, and the administration sites were then cleaned with a warm 0.9% sodium chloride injection solution. A single dose was administered. Potential erythema and edema at the administration sites were examined and recorded for each group of animals prior to administration and at 1 h, 24 hrs, 48 hrs, 72 hrs and 14 days after drug removal. Erythema and edema were recorded and scored according to the scoring criteria. The experiment revealed that all the test gels met the irritation criteria.

Each test gel contained 1.2 wt% 17β-neriifolin, 1.5 wt% sodium carboxymethyl cellulose, 40 wt% anhydrous ethanol, 0.5 wt% to 1.0 wt% penetration enhancer, and 20 wt% glycerol, with the balance being water. The 0.5 wt% to 1.0 wt% penetration enhancers were 1.0% azone, 1.0% borneol, 0.75% borneol, and 0.5% borneol, respectively.

Example 7

The Franz diffusion cell method was used to determine the *in vitro* transdermal absorption, including the transdermal amount, residual amount on the skin surface, and intradermal retention amount.

Transdermal amount: Pig skin (which had been immersed in normal saline for about 30 min before use, and was then taken out and dried with filter paper) was fixed between the receiving cell and dosing cell, with the stratum corneum facing the dosing cell. About 200 mg of the gel of the present application were precisely weighed and evenly applied to the pig skin on the dosing cell side. Eighteen milliliters of 15% ethanol-normal saline preheated to 32 °C±0.5 °C were added to the receiving cell and stirred at a rotation speed of 200 rpm. Then, 0.5 mL of the receiving solution was sampled at 1 h, 2 hrs, 3 hrs, 4 hrs, and 6 hrs (with an equal volume of a blank receiving solution at the same temperature added into the receiving cell at the same time). The samples were centrifuged, and the supernatants were collected and subjected to HPLC analysis. The transdermal amount of the drug was calculated based on the peak area.

Residual amount on the skin surface: After permeation with the formulation of the present application for 6 hrs, the pig skin (ensuring that the gel remaining on the surface was not lost) was rinsed with a total of 25 mL of a saturated sodium chloride solution (5 mL each time for 5 times, vortexed for 0.5 min per wash). The rinsates were collected into a 100-mL volumetric flask, and 25 mL of methanol were added. The mixture was ultrasonicated for extraction for 20 min, diluted to the volume with a 50% methanol-saturated sodium chloride solution, mixed by shaking until uniformity, and then filtered. The subsequent filtrate was collected and subjected to HPLC analysis, and the residual amount on the skin surface was calculated based on the peak area.

Intradermal retention amount: The rinsed pig skin was treated with 25 mL of an 80% methanol-saturated sodium chloride solution, heated in a water bath at 70 °C for 30 min, cut into pieces, and then homogenized. The homogenate was centrifuged (at 4 °C and 14000 rpm for 10 min). The supernatant was collected and filtered. The subsequent filtrate was collected and subjected to HPLC analysis, and the intradermal retention amount was calculated based on the peak area.

The test gel was prepared according to the method in Example 4, containing 0.4 wt% 17β-neriifolin, 1.5 wt% sodium carboxymethyl cellulose, 40 wt% anhydrous ethanol, 0.5 wt% borneol, and 20 wt% glycerol, with the balance being water.

It was found through calculation that within 6 hrs, the drug in the gel of the present application had a transdermal amount of less than 1%, a residual amount on the skin surface of 80%-95%, and an intradermal retention amount of approximately 10%. The results suggest that the drug in the gel prepared by the present disclosure should be substantially retained with the skin tissue, achieving a high local drug concentration, and little would be absorbed into the blood, so that the side effects caused by systemic drug distribution can be reduced.

Example 8

Healthy SPF-grade Hartley guinea pigs (half male and half female) with no back skin injury were selected. A negative control group (white vaseline), a positive control group (2,4-dinitrochlorobenzene vaseline ointment), test substance group I (17β-neriifolin gel, with the content of 17β-neriifolin being 2 mg/g), test substance group II (17β-neriifolin gel, with the content of 17β-neriifolin being 4 mg/g), and test substance group III (17β-neriifolin gel, with the content of 17β-neriifolin being 8 mg/g) were set. For all groups, the dose was 0.5 g of gel/animal/administration, and the administration area on the skin was 2.5 cm × 2.5 cm. The drug in each group was kept in contact with the left dorsal skin of each animal in the corresponding group for 6 hrs to test the sensitivity. Potential skin reactions were observed, recorded, and scored prior to administration and at 1 h and 24 hrs after residual drug removal. The drug administration for sensitivity testing was conducted once on day 1, day 8, and day 15, for a total of three applications. On day 29, the drug was kept in contact with the right-side skin of the animals for 6 hrs. Potential skin reactions were observed and recorded prior to administration and at 24 hrs and 48 hrs after residual drug removal. The test results were assessed.

The test gels of the test substance groups I, II, and III were prepared according to the method in Example 4, each containing 1.5 wt% sodium carboxymethyl cellulose, 40 wt% anhydrous ethanol, 0.5 wt% borneol, and 20 wt% glycerol,

with the balance being water.

The results showed that the gel formulations of the present application did not cause allergic reactions in the skin of guinea pigs, with all allergic reaction results being negative.

Example 9

Healthy New Zealand rabbits (half male and half female) with no back injury were used. A blank gel control group and 17β-neriifolin gel groups (with the contents of 17β-neriifolin being 2 mg/g and 4 mg/g, respectively) were set. Comparative administration was performed by topical application on the left and right sides of the same animal's skin, and the administration sites were covered with patches after application. The test substance was administered to the left side, and white vaseline was administered to the right side as a negative control. The administration area was 3.0 cm × 3.0 cm. The patches on the administration sites were maintained on both sides of the skin for about 4 hrs before removal, and the administration sites were then cleaned with a warm 0.9% sodium chloride injection solution. A single dose was administered. Potential erythema and edema at the administration sites were examined and recorded for each group of animals prior to administration and at 1 h, 24 hrs, 48 hrs, 72 hrs and 14 days after drug removal. The erythema and edema were recorded and scored according to the scoring criteria. Additionally, the left-side skin of the administration sites in each group of animals was subjected to histopathological examination at 72 hrs and 14 days after drug removal.

The results show that after single-dose topical administration on the intact skin of New Zealand rabbits in the blank gel control group and the 17β-neriifolin gel groups (2 mg/g and 4 mg/g), both left and right skin sites of each animal exhibited irritation reaction scores within the non-irritating range, with no abnormalities found in microscopic examination and no test substance-related skin irritation reactions observed.

The test gel was prepared according to the method in Example 4, containing 1.5 wt% sodium carboxymethyl cellulose, 40 wt% anhydrous ethanol, 0.5 wt% borneol, and 20 wt% glycerol, with the balance being water.

Example 10

Clinical studies were conducted to evaluate the efficacy and safety of 17β-neriifolin topically applied to subjects with actinic keratosis. 17β-Neriifolin showed good clinically relevant efficacy in the treatment of actinic keratosis. From the perspectives of local tolerability and local skin reactions (LSR), 17β-neriifolin was shown to be safe.

This study consisted of Phase I (a dose escalation trial) and Phase II (an expansion trial). Phase I was a multicenter, open-label, and dose-escalation trial investigating the safety, tolerability, PK profile, and preliminary efficacy of 17β-neriifolin gel at different dose levels applied to lesions of actinic keratosis. Phase II was a multicenter, open-label trial investigating the efficacy, safety and PK profile of 17β-neriifolin gel at the target dose in subjects with actinic keratosis.

1. Case selection

Patients aged ≥ 18 years were selected. All patients complied with the study protocols and provided informed consent.

2. Inclusion criteria

Inclusion criteria: (1) meeting the diagnostic criteria for AK by *Clinical Dermatology,* and being diagnosed via histopathological examination or noninvasive diagnosis (dermatoscopy, skin CT, etc.); and (2) having complete clinical data.

3. Methods

| Dose Group/Level | Dose based on 17β-neriifolin (mg/cm$^2$) | Dose based on gel (mg/cm$^2$) | |
|---|---|---|---|
| | | Dose(mg/cm$^2$) | Gel specification |
| 1 | 0.008 | 4 | 2 mg/g |
| 2 | 0.016 | 8 | 2 mg/g |
| 3 | 0.027 | 6.75 | 4 mg/g |
| 4 | 0.04 | 10 | 4 mg/g |

(continued)

| Dose Group/Level | Dose based on 17β-neriifolin (mg/cm$^2$) | Dose based on gel (mg/cm$^2$) | |
|---|---|---|---|
| | | Dose(mg/cm$^2$) | Gel specification |
| 5 | 0.06 | 15 | 4 mg/g |

The Phase I trial was a dose escalation study involving single-dose and multiple-dose administrations. After single-dose administration, a 3-day DLT (dose-limiting toxicity) observation was performed. Starting on day 4, multiple-dose administration began, with the drug administered once every other day for three consecutive weeks.
Method of administration: Evenly apply the gel to the lesions, avoiding rinsing and rubbing. The application site was then covered with a breathable waterproof dressing and secured with non-irritating adhesive tape. It was ensured that the drug was kept in contact with the skin for 4±0.5 h per administration. To remove the drug, the administration site was rinsed and wiped with room-temperature normal saline.

Calculation of administration dose: administration area (cm$^2$) = (radius of affected area)$^2$ × π

Dose (gel, mg) = [dose (gel amount) per cm$^2$ in the current dose group] × administration area

Conversion formula between mg and microliter: gel weight ÷ gel density = volume (in microliters, μL)

Administration area: 2 treatment units (1 treatment unit was defined as the area of one index finger knuckle of each subject). The total administration area of the target lesions should not exceed 25 cm$^2$.
During the hospitalization period, medication was administered under the supervision of a physician or nurse. The dose and time of administration were recorded in the subject's diary card. During the non-hospitalization period, the study physician and nurse instructed the subjects to apply the medication in a fixed time period each day according to the above-specified administration method. The specific administration regimen is detailed in the standard operating procedures for drug use.
The frequency of administration and intermission periods of the study drug may be appropriately adjusted based on human safety, PK data, and efficacy.

4. Observation indicators

Evaluation of the clinical effects.

Efficacy evaluation indicators applicable to subjects with actinic keratosis:

Complete clearance rate: refers to the proportion of subjects who achieve 100% clearance of the lesions;
Partial clearance rate: the proportion of subjects who achieve at least 75% clearance of the lesions;
Partial clearance rate: the proportion of subjects who achieve at least 50% clearance of the lesions;
Specific clearance rate of lesions: the proportion of subjects with a reduction in the number of cleared lesions from baseline to the assessment time;
Dermatology quality of life index: the SF-36 rating scale was used to evaluate the subject's quality of life;
Safety indicators: adverse events (including local reactions at administration sites), physical examinations, vital signs, laboratory examinations (including blood routine examination, blood biochemistry, urinalysis, etc.), electrocardiograms, echocardiogram, etc.

5. **Summary of safety and efficacy data for subjects enrolled in Phase I**

A total of 29 subjects were enrolled in this study. Demographic information of the subjects is shown in the table below.

**Demographic data summary - all enrolled subjects (China and Australia)**

| | 17 β -Neriifolin (N = 29) |
|---|---|
| **Age (years)** | |
| n (number of subjects) | 29 |
| Mean (SD) | 72.6 (11.70) |
| Median | 75.0 (67.0,80.0) |
| Min; Max | 32; 91 |
| **Sex. n (%)** | |
| n (number of subjects) | 29 |
| Male | 10 (34.5%) |
| Female | 19 (65.5%) |
| **Ethnicity, n (%)** | |
| n (number of subjects) | 29 |
| Han Chinese | 23 (79.3%) |
| Others | 6 (20.7) |
| **ECOG score, n (%)** | |
| n (number of subjects) | 29 |
| 0 | 27 (93.1%) |
| 1 | 2 (6.9%) |
| 2 | 0 |
| 3 | 0 |
| 4 | 0 |
| 5 | 0 |

**Safety results**

Overall, 29 subjects received at least one application of 17β-neriifolin topical gel. There were five administration dose levels/groups (0.008 mg/cm$^2$, 0.016 mg/cm$^2$, 0.027 mg/cm$^2$, 0.04 mg/cm$^2$, and 0.06 mg/cm$^2$). All groups showed relatively good safety profiles. Regarding the safety profiles, the 17β-neriifolin topical gel showed similar safety profiles in Chinese and Australian subjects. According to the investigators' evaluation, the drug-related adverse events were primarily reactions at the skin application sites.

A total of 20 (69%) subjects experienced treatment-related adverse events (TRAEs) that the investigators assessed as drug-related. The most frequently reported TRAEs were skin pruritus (7/29, 24.1%), skin desquamation (5/29, 17.2%), and skin crusting (5/29, 17.2%). These were mainly mild local reactions at the administration sites. All AEs were Grade 1 or 2 according to the CTCAE, and no Grade 3 or higher TEAEs occurred.

There were no SAEs, and no DLTs were observed.

No fatal AEs were reported.

In summary, the safety profiles of 17β-neriifolin gel were similar in Chinese and Australian subjects. According to the investigators' evaluation, the drug-related adverse events were primarily local reactions at the skin application sites, such as pruritus, desquamation, crusting, and the like, all of which were Grade 1-2 mild reactions, indicating relatively good overall safety.

**Efficacy results:**

According to the safety, tolerability, pharmacokinetic, and efficacy data generated in this clinical study, the efficacy-evaluable subjects in dose group 5 (China and Australia) included nine cases of actinic keratosis.

In dose group 5 of this study (China and Australia), a total of nine subjects with actinic keratosis were enrolled and were efficacy-evaluable on D16, on D24, and on day 28 of the follow-up period after the last dose. The investigators' assessment results show that: after treatment with 17β-neriifolin topical gel, the number of subjects with actinic keratosis

who achieved complete clearance of individual lesions was five (55.56%).

Proportion of individual lesion clearance in actinic keratosis treated with 17β-neriifolin (0.06 mg/cm$^2$)

| Proportion of individual lesion clearance | D16 n(%) | D24 n (%) | On day 28 of the follow-up period after the last dose n (%) |
|---|---|---|---|
| 100% | 1/9 (11.11%) | 2/9 (22.22%) | 5/9 (55.56%) |

"n" is the number of subjects.

**Pharmacokinetic study results:**

Serum concentrations of 17β-neriifolin in the Chinese and Australian patients treated with the topical gel were measured and analyzed during the dose-escalation phase (dose levels 1-5) of the clinical study.

Serum concentrations of 17β-neriifolin were all determined using liquid chromatography-mass spectrometry (LC/MS/MS), with the lower limit of quantification (LLOQ) being 0.02 ng/mL. No dose-dependent increase in the systemic exposure of 17β-neriifolin was observed within a 0.008-0.06 mg/cm$^2$ dose range. Due to the low overall systemic absorption rate and sparse data, a statistically significant pharmacokinetic evaluation could not be performed (only seven Chinese subjects had plasma drug concentrations above the LLOQ and in the concentration range of 0.022-0.106 ng/mL, while a total of four Australian subjects had plasma drug concentrations above the LLOQ and in the concentration range of 0.021-0.101 ng/mL).

It can be seen that the gel of the present application exhibited good therapeutic efficacy and was confirmed to have good safety.

In the description of this specification, the description of reference terms "one embodiment", "some embodiments", "an example", "a specific example", or "some examples", and the like means that a particular feature, structure, material, or characteristic described in connection with the embodiments or examples is included in at least one embodiment or example of the present application. In this specification, the schematic descriptions of the terms described above do not necessarily refer to the same embodiment or example. Furthermore, the specific features, structures, materials, or characteristics described herein may be combined in any suitable manner in any one or more embodiments or examples. In addition, in the absence of contradiction, those skilled in the art can combine the different embodiments or examples described in this specification or combine the features of different embodiments or examples.

Although the examples of the present application have been shown and described above, it will be appreciated that the above examples are exemplary and should not be construed as limiting the present application, and that modifications, substitutions, and alterations can be made to the above examples by those of ordinary skill in the art within the scope of the present application.

## Claims

1. A compound, which is 17β-neriifolin or a pharmaceutically acceptable salt, racemate, stereoisomer, enantiomer, diastereomer, tautomer, metabolite, solvate or prodrug thereof, for use in the treatment and/or prevention of actinic keratosis via topical administration.

2. A method for treating and/or preventing actinic keratosis, comprising administering 17β-neriifolin or a pharmaceutically acceptable salt, racemate, stereoisomer, enantiomer, diastereomer, tautomer, metabolite, solvate or prodrug thereof.

3. The method according to Claim 2, **characterized in that** the method comprises administering to a subject in need thereof a therapeutically effective amount of 17β-neriifolin or a pharmaceutically acceptable salt, racemate, stereoisomer, enantiomer, diastereomer, tautomer, metabolite, solvate or prodrug thereof.

4. The method according to any one of the preceding claims, **characterized in that** 17β-neriifolin or a pharmaceutically acceptable salt, racemate, stereoisomer, enantiomer, diastereomer, tautomer, metabolite, solvate or prodrug thereof is administered to the affected area(s) of the subject at a dose of: about 0.0003 mg to about 100 mg; about 0.008 mg to

about 90 mg; about 0.1 mg to about 80 mg; about 0.1 mg to about 70 mg; about 0.1 mg to about 60 mg; about 0.1 mg to about 50 mg; about 0.1 mg to about 40 mg; about 0.1 mg to about 30 mg; about 0.1 mg to about 20 mg; about 0.1 mg to about 15 mg; about 0.1 mg to about 10 mg; about 0.1 mg to about 8 mg; about 0.2 mg to about 5 mg; about 0.5 mg to about 2.5 mg; about 0.02 mg, about 0.05 mg, about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3 mg, about 4 mg, or about 5 mg.

**5.** The method according to any one of the preceding claims, **characterized in that** 17β-neriifolin or a pharmaceutically acceptable salt, racemate, stereoisomer, enantiomer, diastereomer, tautomer, metabolite, solvate or prodrug thereof is administered to the affected area(s) of the subject at a dose of: about 0.0003 mg/cm$^2$ to about 10 mg/cm$^2$; preferably about 0.001 mg/cm$^2$ to about 1 mg/cm$^2$; preferably about 0.002 mg/cm$^2$ to about 0.5 mg/cm$^2$; preferably about 0.004 mg/cm$^2$ to about 0.25 mg/cm$^2$; preferably about 0.008 mg/cm$^2$ to about 0.2 mg/cm$^2$; preferably about 0.01 mg/cm$^2$ to about 0.18 mg/cm$^2$; preferably about 0.016 mg/cm$^2$ to about 0.16 mg/cm$^2$; preferably about 0.02 mg/cm$^2$ to about 0.15 mg/cm$^2$; preferably about 0.025 mg/cm$^2$ to about 0.14 mg/cm$^2$; preferably about 0.027 mg/cm$^2$ to about 0.13 mg/cm$^2$; preferably about 0.03 mg/cm$^2$ to about 0.125 mg/cm$^2$; preferably about 0.035 mg/cm$^2$ to about 0.12 mg/cm$^2$; preferably about 0.04 mg/cm$^2$ to about 0.1 mg/cm$^2$; about 0.001 mg/cm$^2$, about 0.002 mg/cm$^2$, about 0.003 mg/cm$^2$, about 0.004 mg/cm$^2$, about 0.005 mg/cm$^2$, about 0.006 mg/cm$^2$, about 0.007 mg/cm$^2$, about 0.008 mg/cm$^2$, about 0.009 mg/cm$^2$, about 0.01 mg/cm$^2$, about 0.015 mg/cm$^2$, about 0.02 mg/cm$^2$, about 0.025 mg/cm$^2$, about 0.03 mg/cm$^2$, about 0.035 mg/cm$^2$, about 0.04 mg/cm$^2$, about 0.045 mg/cm$^2$, about 0.05 mg/cm$^2$, about 0.055 mg/cm$^2$, about 0.06 mg/cm$^2$, about 0.065 mg/cm$^2$, about 0.07 mg/cm$^2$, about 0.075 mg/cm$^2$, about 0.08 mg/cm$^2$, about 0.085 mg/cm$^2$, about 0.09 mg/cm$^2$, about 0.095 mg/cm$^2$, about 0.1 mg/cm$^2$, about 0.15 mg/cm$^2$, about 0.2 mg/cm$^2$, about 0.25 mg/cm$^2$, about 0.3 mg/cm$^2$, about 0.35 mg/cm$^2$, about 0.4 mg/cm$^2$, about 0.45 mg/cm$^2$, about 0.5 mg/cm$^2$, about 0.55 mg/cm$^2$, about 0.6 mg/cm$^2$, or about 0.65 mg/cm$^2$.

**6.** The method according to any one of the preceding claims, **characterized in that** the affected area(s) of the subject is/are about 0.01 cm$^2$ to about 300 cm$^2$; preferably, the affected area(s) of the subject is/are: about 1 cm$^2$ to about 200 cm$^2$, about 1 cm$^2$ to about 100 cm$^2$, about 1 cm$^2$ to about 75 cm$^2$, about 1 cm$^2$ to about 50 cm$^2$, or about 1 cm$^2$ to about 25 cm$^2$; about 10 cm$^2$ to about 200 cm$^2$, about 10 cm$^2$ to about 100 cm$^2$, about 10 cm$^2$ to about 75 cm$^2$, about 10 cm$^2$ to about 50 cm$^2$, or about 10 cm$^2$ to about 25 cm$^2$; about 25 cm$^2$ to about 200 cm$^2$, about 25 cm$^2$ to about 100 cm$^2$, or about 25 cm$^2$ to about 75 cm$^2$; about 25 cm$^2$ to about 90 cm$^2$, about 25 cm$^2$ to about 80 cm$^2$, or about 25 cm$^2$ to about 70 cm$^2$, about 25 cm$^2$ to about 60 cm$^2$, about 25 cm$^2$ to about 50 cm$^2$, about 25 cm$^2$ to about 40 cm$^2$, or about 25 cm$^2$ to about 30 cm$^2$; preferably, the affected area(s) of the subject is/are about 0.2 cm$^2$, about 0.5 cm$^2$, about 1 cm$^2$, about 2 cm$^2$, about 3 cm$^2$, about 4 cm$^2$, about 5 cm$^2$, about 6 cm$^2$, about 7 cm$^2$, about 8 cm$^2$, about 9 cm$^2$, about 10 cm$^2$, about 11 cm$^2$, about 12 cm$^2$, about 13 cm$^2$, about 14 cm$^2$, about 15 cm$^2$, about 16 cm$^2$, about 17 cm$^2$, about 18 cm$^2$, about 19 cm$^2$, about 20 cm$^2$, about 25 cm$^2$, about 30 cm$^2$, about 35 cm$^2$, about 40 cm$^2$, about 45 cm$^2$, about 50 cm$^2$, about 55 cm$^2$, about 60 cm$^2$, about 65 cm$^2$, about 70 cm$^2$, about 75 cm$^2$, about 80 cm$^2$, about 85 cm$^2$, about 90 cm$^2$, about 95 cm$^2$, or about 100 cm$^2$.

**7.** The method according to any one of the preceding claims, **characterized in that** the affected area(s) of the subject is/are on the skin.

**8.** The method according to any one of the preceding claims, **characterized in that** the affected area(s) of the subject is/are located at one or more positions independently selected from the scalp, forehead, forearm, face, nose, ears, eyelids, lips, neck, arms, hands, trunk, legs, and feet.

**9.** The method according to any one of the preceding claims, **characterized in that** 17β-neriifolin or a pharmaceutically acceptable salt, racemate, stereoisomer, enantiomer, diastereomer, tautomer, metabolite, solvate or prodrug thereof is formulated into a pharmaceutical composition suitable for topical use.

**10.** The method according to any one of the preceding claims, **characterized in that** the content of 17β-neriifolin or a pharmaceutically acceptable salt, racemate, stereoisomer, enantiomer, diastereomer, tautomer, metabolite, solvate or prodrug thereof is about 0.025 wt% to 4 wt%; preferably about 0.05 wt% to 3 wt%; preferably about 0.2 wt% to 0.8 wt%; preferably about 0.075 wt%, about 0.1 wt%, about 0.125 wt%, about 0.15 wt%, about 0.175 wt%, about 0.2 wt%, about 0.225 wt%, about 0.25 wt%, about 0.275 wt%, about 0.3 wt%, about 0.325 wt%, about 0.35 wt%, about 0.375 wt%, about 0.4 wt%, about 0.425 wt%, about 0.45 wt%, about 0.475 wt%, about 0.5 wt%, about 0.525 wt%, about 0.55 wt%, about 0.575 wt%, about 0.6 wt%, about 0.625 wt%, about 0.65 wt%, about 0.675 wt%, about 0.7 wt%, about

0.725 wt%, about 0.75 wt%, about 0.775 wt%, about 0.8 wt%, about 0.825 wt%, about 0.85 wt%, about 0.875 wt%, about 0.9 wt%, about 0.925 wt%, about 0.95 wt%, about 0.975 wt%, about 1 wt%, about 1.25 wt%, about 1.5 wt%, about 1.75 wt%, about 2.0 wt%, about 2.25 wt%, about 2.5 wt%, or about 2.75 wt%, based on the total weight of the pharmaceutical composition.

**11.** The method according to any one of the preceding claims, **characterized in that** the amount of the pharmaceutical composition administered to an affected skin area(s) ranges/range from about 0.001 g/cm$^2$ of skin surface area to about 0.5 g/cm$^2$ of skin surface area; preferably about 0.0015 g/cm$^2$ of skin surface area to about 0.2 g/cm$^2$ of skin surface area; preferably about 0.0015 g/cm$^2$ of skin surface area to about 0.15 g/cm$^2$ of skin surface area; preferably about 0.002 g/cm$^2$ of skin surface area to about 0.125 g/cm$^2$ of skin surface area; preferably about 0.003 g/cm$^2$ of skin surface area to about 0.1 g/cm$^2$ of skin surface area; preferably about 0.004 g/cm$^2$ of skin surface area; about 0.005 g/cm$^2$ of skin surface area; about 0.006 g/cm$^2$ of skin surface area; about 0.007 g/cm$^2$ of skin surface area; about 0.008 g/cm$^2$ of skin surface area; about 0.009 g/cm$^2$ of skin surface area; about 0.01 g/cm$^2$ of skin surface area; about 0.0125 g/cm$^2$ of skin surface area; about 0.015 g/cm$^2$ of skin surface area; about 0.0175 g/cm$^2$ of skin surface area; about 0.02 g/cm$^2$ of skin surface area; about 0.03 g/cm$^2$ of skin surface area; about 0.04 g/cm$^2$ of skin surface area; about 0.05 g/cm$^2$ of skin surface area; about 0.06 g/cm$^2$ of skin surface area; about 0.07 g/cm$^2$ of skin surface area; about 0.08 g/cm$^2$ of skin surface area; or about 0.09 g/cm$^2$ of skin surface area.

**12.** The method according to any one of the preceding claims, **characterized in that** 17β-neriifolin or a pharmaceutically acceptable salt, racemate, stereoisomer, enantiomer, diastereomer, tautomer, metabolite, solvate or prodrug thereof is administered daily, at one-day intervals, at two-day intervals, at three-day intervals, at four-day intervals, at five-day intervals, at six-day intervals, or at seven-day intervals; generally, on the day of administration, one, two, three, or four administrations are performed.

**13.** The method according to any one of the preceding claims, **characterized in that** the pharmaceutical composition is a gel.

**14.** The method according to any one of the preceding claims, **characterized in that** the gel comprises a gel matrix, wherein the gel matrix is preferably selected from at least one of sodium carboxymethyl cellulose, carbomer, gelatin, and alginate; the content of the gel matrix is about 0.5 wt% to 5 wt%; preferably about 1 wt% to 4 wt%; preferably about 1 wt% to 3 wt%; preferably about 1.5 wt%, about 2 wt%, about 2.5 wt%, or about 3 wt%, based on the total weight of the pharmaceutical composition; and/or

the gel comprises a humectant, wherein the humectant is selected from at least one of alcoholic humectants; preferably, said humectant is glycerol, wherein the content of the humectant is about 5 wt% to 35 wt%; preferably about 10 wt% to 30 wt%; preferably about 15 wt% to 25 wt%; more preferably about 15 wt%, about 20 wt%, about 25 wt%, or about 30 wt%, based on the total weight of the pharmaceutical composition; and/or
the gel comprises a solvent; preferably, the solvent is selected from at least one of ethanol, propylene glycol, and benzyl alcohol; more preferably, the ethanol is 95% ethanol or anhydrous ethanol; the content of the solvent is about 20 wt% to 75 wt%; preferably about 25 wt% to 70 wt%; more preferably about 30 wt%, about 35 wt%, about 40 wt%, about 45 wt%, about 50 wt%, about 55 wt%, about 60 wt%, or about 65 wt%, based on the total weight of the pharmaceutical composition; and/or
the gel comprises water, preferably purified water, distilled water, or deionized water; the content of the water is about 20 wt% to 75 wt%; preferably about 25 wt% to 70 wt%; more preferably about 30 wt%, about 35 wt%, about 40 wt%, about 45 wt%, about 50 wt%, about 55 wt%, about 60 wt%, or about 65 wt%, based on the total weight of the pharmaceutical composition; and/or
the gel comprises a penetration enhancer, wherein the penetration enhancer is selected from at least one of azone, borneol, Tween 80, sodium dodecyl sulfate, and poloxamer; more preferably, the penetration enhancer is azone or borneol; the content of the penetration enhancer is about 0 wt% to 5 wt%; preferably about 0.1 wt% to 4 wt%; more preferably about 0.25 wt%, about 0.5 wt%, about 0.75 wt%, about 1 wt%, about 1.25 wt%, about 1.5 wt%, about 1.75 wt%, about 2 wt%, about 2.25 wt%, about 2.5 wt%, about 2.75 wt%, about 3 wt%, about 3.25 wt%, about 3.5 wt%, or about 3.75 wt%, based on the total weight of the pharmaceutical composition.

**15.** The method according to any one of the preceding claims, **characterized in that** the pharmaceutical composition comprises a preservative; the preservative is a paraben preservative and/or butylated hydroxytoluene; preferably, the paraben preservative is methylparaben and/or ethylparaben; the content of the preservative is about 0 wt% to 4 wt%; preferably about 0.1 wt% to 3 wt%; more preferably about 0.25 wt%, about 0.5 wt%, about 0.75 wt%, about 1 wt%, about 1.25 wt%, about 1.5 wt%, about 1.75 wt%, about 2 wt%, about 2.25 wt%, about 2.5 wt%, or about 2.75 wt%,

based on the total weight of the pharmaceutical composition.

**16.** The method according to any one of the preceding claims, **characterized in that** the pharmaceutical composition comprises:

about 0.2 wt% to 0.8 wt% 17β-neriifolin or a pharmaceutically acceptable salt, racemate, stereoisomer, enantiomer, diastereomer, tautomer, metabolite, solvate or prodrug thereof; and/or
about 1.5 wt% gel matrix; and/or
about 40 wt% solvent; and/or
about 0.5 wt% penetration enhancer; and/or
about 20 wt% humectant;
the balance being water.

**17.** The method according to any one of the preceding claims, **characterized in that** compared to other treatments for actinic keratosis, the administration of 17β-neriifolin or a pharmaceutically acceptable salt, racemate, stereoisomer, enantiomer, diastereomer, tautomer, metabolite, solvate or prodrug thereof reduces the number and/or severity of local skin reactions or additional adverse side effects in the subject.

**18.** The method according to any one of the preceding claims, **characterized in that** the local skin reaction is selected from vesicle formation, pustule formation, erosion, ulceration, redness, swelling, scaling, desquamation, induration, dryness, pus, and blistering.

## INTERNATIONAL SEARCH REPORT

International application No.
**PCT/CN2024/124481**

**A. CLASSIFICATION OF SUBJECT MATTER**

C07J 19/00(2006.01)i； A61K 31/56(2006.01)i； A61P 17/00(2006.01)i

According to International Patent Classification (IPC) or to both national classification and IPC

**B. FIELDS SEARCHED**

Minimum documentation searched (classification system followed by classification symbols)

IPC： C07J， A61K， A61P

Documentation searched other than minimum documentation to the extent that such documents are included in the fields searched

Electronic data base consulted during the international search (name of data base and, where practicable, search terms used)

VCN, CNTXT, WPABS, WPABSC, ENTXT, ENTXTC, DWPI, VEN, CJFD, REGISTRY, CAPLUS 依据申请人和发明人进行了追踪, track according to applicants and inventors, 依据本申请的活性化合物进行了检索, search according to active compounds of the present application, 黄夹次甙乙 or Neriifolin, 日光性角化, 光, 角化, actinic keratosis

**C. DOCUMENTS CONSIDERED TO BE RELEVANT**

| Category* | Citation of document, with indication, where appropriate, of the relevant passages | Relevant to claim No. |
|---|---|---|
| X | CN 108948119 A (ENZHI (GUANGZHOU) PHARMACEUTICALS LIMITED et al.) 07 December 2018 (2018-12-07)<br>description, page 1, paragraph 5 | 1 |
| A | WO 2018165647 A1 (ATHENEX INC.) 13 September 2018 (2018-09-13)<br>embodiments 1-6, tables 1-13, and figures 1-5 | 1-18 |
| A | CN 109908160 A (TIANJIN UNIVERSITY OF TRADITIONAL CHINESE MEDICINE) 21 June 2019 (2019-06-21)<br>entire document | 1-18 |
| A | CN 110891575 A (ATHENEX INC.) 17 March 2020 (2020-03-17)<br>entire document | 1-18 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 January 2025** | **09 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/124481

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **2-18**
because they relate to subject matter not required to be searched by this Authority, namely:

Claims 2-18 relate to a treatment method implemented on the human or animal body (PCT Rule 39.1(iv)). Nevertheless, a search is still performed on the basis of the technical subject matter of the use of the compound/composition in the preparation of a corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/124481**

| Patent document cited in search report | Publication date (day/month/year) | Patent family member(s) | Publication date (day/month/year) |
|---|---|---|---|
| CN 108948119 A | 07 December 2018 | EP 3625242 A1 | 25 March 2020 |
| | | EP 3625242 A4 | 07 July 2021 |
| | | US 2020157139 A1 | 21 May 2020 |
| | | US 11091510 B2 | 17 August 2021 |
| | | WO 2018210252 A1 | 22 November 2018 |
| | | EP 4335513 A2 | 13 March 2024 |
| | | EP 4335513 A3 | 08 May 2024 |
| | | CA 3100875 A1 | 22 November 2018 |
| WO 2018165647 A1 | 13 September 2018 | None | |
| CN 109908160 A | 21 June 2019 | None | |
| CN 110891575 A | 17 March 2020 | CA 3055938 A1 | 13 September 2018 |
| | | MX 2021013354 A | 18 November 2021 |
| | | JP 2023015269 A | 31 January 2023 |
| | | KR 20240090837 A | 21 June 2024 |
| | | AU 2018231144 A1 | 03 October 2019 |
| | | AU 2018231144 B2 | 21 December 2023 |
| | | JP 2020510042 A | 02 April 2020 |
| | | JP 7502863 B2 | 19 June 2024 |
| | | AU 2024201828 A1 | 02 May 2024 |
| | | TW 201842913 A | 16 December 2018 |
| | | TWI 841523 B | 11 May 2024 |
| | | BR 112019018687 A2 | 07 April 2020 |
| | | EP 3592355 A1 | 15 January 2020 |
| | | EP 3592355 A4 | 06 January 2021 |
| | | US 2018256589 A1 | 13 September 2018 |
| | | US 10617693 B2 | 14 April 2020 |
| | | US 2020197405 A1 | 25 June 2020 |
| | | US 11497750 B2 | 15 November 2022 |
| | | IL 269182 A | 28 November 2019 |
| | | IL 269182 B1 | 01 July 2023 |
| | | IL 269182 B2 | 01 November 2023 |
| | | WO 2018165647 A1 | 13 September 2018 |
| | | MX 2019010707 A | 15 January 2020 |
| | | US 2023172942 A1 | 08 June 2023 |
| | | RU 2019131757 A | 12 April 2021 |
| | | RU 2019131757 A3 | 31 May 2021 |
| | | ZA 202100106 B | 31 August 2022 |
| | | KR 20190141661 A | 24 December 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311331585
- CN 104736157 A